# EUROPÄISCHE PATENTSCHRIFT

(11) **EP 2 268 786 B1**
(45) Veröffentlichungstag und Bekanntmachung des Hinweises auf die Patenterteilung: **16.05.2012**
(21) Anmeldenummer: 09714319.2
(22) Anmeldetag: 17.02.2009
(51) Int. Cl.: C12M 1/00, C12M 1/02

(54) **INKUBATOR MIT SCHÜTTELVORRICHTUNG**
INCUBATOR COMPRISING A SHAKING DEVICE
INCUBATEUR À DISPOSITIF AGITATEUR

(30) Priorität: 25.02.2008 DE 102008010780
(43) Veröffentlichungstag der Anmeldung: 05.01.2011
(73) Patentinhaber: Sartorius Stedim Biotech GmbH, 37079 Göttingen (DE)
(72) Erfinder: BAUMFALK, Reinhard, 37083 Göttingen (DE); OBERMANN, Stefan, 37139 Adelebsen-Erbsen (DE); SANDROCK, Rainer, 34134 Kassel (DE)
(86) Internationale Anmeldenummer: PCT/EP2009/001089
(87) Internationale Veröffentlichungsnummer: WO 2009/106248

(56) Entgegenhaltungen:
- EP-A- 1 626 082
- EP-A- 1 854 871
- WO-A-02/101000
- DE-A1-102004 052 156
- DE-C1- 19 814 013
- DE-U1-202007 005 865
- US-A- 3 601 372
- US-A- 5 577 837

## Beschreibung

Die Erfindung betrifft einen Inkubator mit einem Inkubationsraum, einer von einem Antrieb antreibbaren Schüttelvorrichtung zum Schütteln von in dem Inkubationsraum anordenbaren Gefäßen mit Zellkulturen, und einem dem Inkubationsraum benachbarten Vorrichtungsraum zur Aufnahme mindestens von Teilen der in den Inkubationsraum hineinragenden Schüttelvorrichtung.

Inkubatoren werden dazu verwendet, in ihrem Inkubationsraum in Gefäßen angeordnete Zellkulturen zu kultivieren. Um ein besseres Zellwachstum zu erreichen, werden dazu Schüttelvorrichtungen verwendet.

Aus der EP 1 626 082 B1 ist ein Inkubator bekannt, der einen Inkubationsraum aufweist, dem an seinem unteren Ende ein Vorrichtungsraum vorgelagert ist. In dem Vorrichtungsraum ist eine Schüttelvorrichtung zum Schütteln bzw. Rütteln von in dem Inkubationsraum angeordneten Gefäßen mit Zellkulturen angeordnet. Zu diesem Zweck ragt eine Achse, an derem freien Ende ein Schütteltisch zur Aufnahme der Gefäße angeordnet ist, in den Inkubationsraum hinein. Die in den Inkubationsraum hineinreichende Achse muss zum einen rotieren und in einer horizontalen Ebene exzentrische hin- und hergehende Bewegungen ausführen.

Nachteilig dabei ist, dass die Abdichtung zwischen Inkubationsraum und Vorrichtungsraum wegen der Schüttelbewegung nur über einen elastischen Faltenbalg möglich ist. Derartige Faltenbalge sind zum einen anfällig gegen Verschleiß und können zum Beispiel einreißen, so dass Feuchtigkeit aus dem Inkubationsraum in den Vorrichtungsraum eindringen kann. Zum anderen kann die im Vorrichtungsraum insbesondere durch den Motor der Schüttelvorrichtung entstehende Wärme über den Faltenbalg das Klima im Inkubationsraum beeinflussen.

Weiterhin ist aus der DE 198 14 013 C1 eine Schüttelvorrichtung bekannt, die als Ganzes in einem Inkubationsraum eines Inkubators angeordnet wird.

Nachteilig bei dieser bekannten Vorrichtung ist, dass die von dieser Vorrichtung abgegebene Wärme das Klima im Inkubationsraum in unerwünschter Weise beeinflussen kann. Auch bei dieser Vorrichtung kann Feuchtigkeit aus dem Inkubationsraum in die Schüttelvorrichtung eindringen.

Weiterhin ist aus der DE 20 2007 005 865 U1 ein Inkubator mit einem Inkubationsraum bekannt, in dem eine Schüttelvorrichtung angeordnet ist. Das Problem der von der Schüttelvorrichtung abgegebenen Wärme in den Inkubationsraum wird bei dieser bekannten Vorrichtung durch einen Kühlkreislauf gelöst. Soweit der Kühlkreislauf aus Kultivierungsgründen nicht ohnehin notwendig ist, ist er nur zur Aufnahme der von der Schüttelvorrichtung abgegebenen Wärme relativ teuer und aufwendig.

Aufgabe der vorliegenden Erfindung ist es daher, einen Inkubator mit einer Schüttelvorrichtung zur Verfügung zu stellen, bei dem das Klima im Inkubationsraum weitgehend unbeeinflusst von einem Wärmeeintrag durch die Schüttelvorrichtung ist. Dabei soll die Schüttelvorrichtung gleichzeitig weitgehend von der Feuchtigkeit des Inkubationsraumes geschützt werden.

Die Aufgabe wird in Verbindung mit dem Oberbegriff des Anspruches 1 dadurch gelöst, dass die Schüttelvorrichtung eine Grundplatte aufweist, die den Inkubationsraum gegenüber dem Vorrichtungsraum abdichtet und auf ihrer dem Inkubationsraum zugewandten Innenseite einen von einem Antriebsarm in einer horizontalen Ebene beweglichen Schütteltisch aufweist, und dass auf der der Innenseite abgewandten Außenseite der Grundplatte ein Motor angeordnet ist, über den eine in der Grundplatte rotierend gelagerte Antriebsachse antreibbar ist, die mit dem Antriebsarm über zwei Achszapfen (20, 30) verbunden ist.

Durch die Anordnung des Motors auf der Außenseite der Grundplatte und des Antriebsarmes mit dem beweglichen Schütteltisch auf der Innenseite der Grundplatte ist es möglich, die Antriebsachse rotierend aber fest, d.h. ohne seitliche Bewegungsüberlagerungen an der Grundplatte zu lagern. Dadurch kann auf einen Faltenbalg verzichtet werden und eine wirksame Abdichtung des Inkubationsraums gegenüber dem Vorrichtungsraum durch die Grundplatte erreicht werden. Dadurch kann ein Wärmeeintrag aus dem Vorrichtungsraum in den Inkubationsraum praktisch vermieden werden. Weiterhin kann ein Feuchteeintrag aus dem Inkubationsraum in den Vorrichtungsraum vermieden werden. Die Schüttelbewegungen des in einer horizontalen Ebene beweglichen Schütteltisches werden auf der dem Inkubationsraum zugewandten Innenseite der Grundplatte durch entsprechende Bewegungen des Antriebsarmes erzeugt. Die Schüttelvorrichtung kann relativ einfach und kostengünstig als Einbauvorrichtung durch Fixieren der Grundplatte zwischen Inkubationsraum und Vorrichtungsraum montiert werden.

Nach einer bevorzugten Ausführungsform der Erfindung weist die Antriebsachse an ihrem dem Vorrichtungsraum zugewandten Ende eine Schwungscheibe auf, die von dem Motor antreibbar ist. Die Schwungscheibe ist dabei zentrisch an einem unteren Achszapfen angeordnet und über einen Antriebsriemen mit einem Antriebsrad des Motors verbunden. Dabei ist an der Schwungscheibe ein radial verschiebbares Kontergewicht angeordnet. Eine durch die zu schüttelnde Masse hervorgerufene Zentrifugalkraft kann durch das Kontergewicht dabei kompensiert werden.

Nach einer weiteren bevorzugten Ausführungsform der Erfindung weist die Antriebsachse an ihrem dem Inkubationsraum zugewandten Ende, also auf der Innenseite der Grundplatte, ein Exzenterstück mit einem oberen Achszapfen auf, der parallel versetzt zu dem unteren Achszapfen angeordnet ist. Somit ist die Antriebsachse über das Exzenterstück mit dem Antriebsarm verbunden. Der Antriebsarm ist zusätzlich mit zwei radial abstehenden Enden über jeweils ein exzentrisches Drehgelenk an der Grundplatte gelagert. Durch Rotation der Antriebsachse wird somit der Antriebsarm in einer quer zur Längsachse der Antriebsachse liegenden Ebene in exzentrischen Bahnen in Schüttel- bzw. Rüttelbewegungen versetzt. Zur Vermeidung von Vibrationen weisen die exzentrischen Drehgelenke dabei jeweils ein elastisches Dämpfungsglied auf.

Nach einer weiteren bevorzugten Ausführungsform der Erfindung ist die Antriebsachse über zwei übereinander angeordnete Kugellager mit der Grundplatte verbunden. Grundsätzlich ist es aber auch möglich, nur ein Kugel- bzw. Wälzlager oder auch ein anderes Gleitlager zu verwenden.

Gemäß einer weiteren bevorzugten Ausführungsform der Erfindung ist zwischen der Antriebsachse und der Grundplatte eine Dichtung angeordnet, die beispielsweise als eine reibungsarme Lippen- oder Labyrinthdichtung ausgebildet ist. Dadurch wird vermieden, dass Feuchtigkeit aus dem Inkubatorraum in den Vorrichtungsraum eindringt.

Weitere Merkmale der Erfindung ergeben sich aus der nachfolgenden ausführlichen Beschreibung und den beigefügten Zeichnungen, in denen bevorzugte Ausführungsformen der Erfindung beispielhaft veranschaulicht sind.

In den Zeichnungen zeigen:

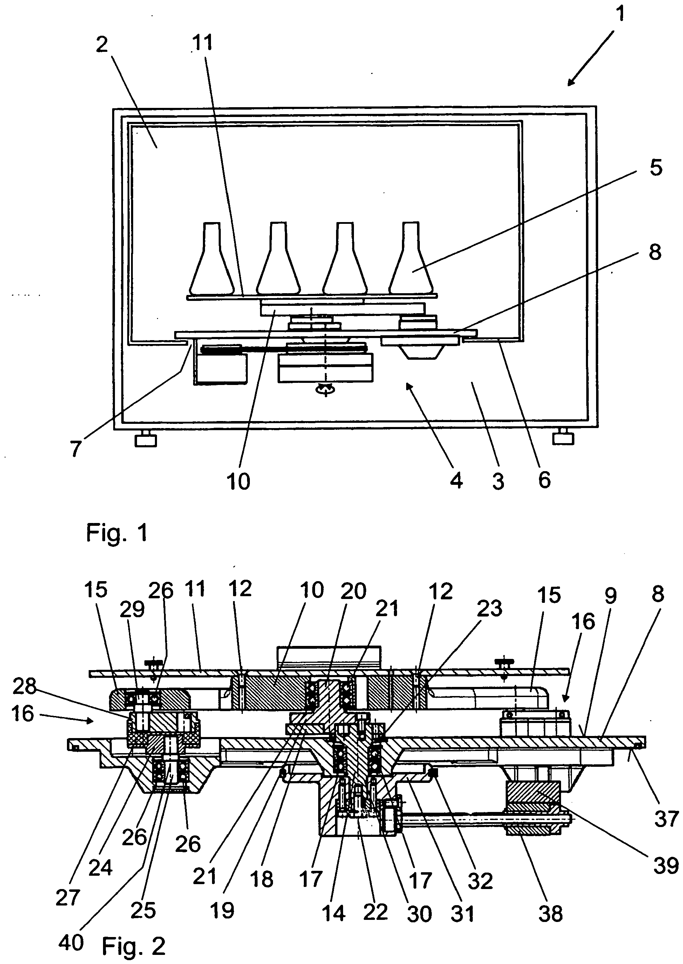

- Figur 1:: eine Seitenansicht einer schematischen Darstellung eines Inkubators mit einer Schüttelvorrichtung,

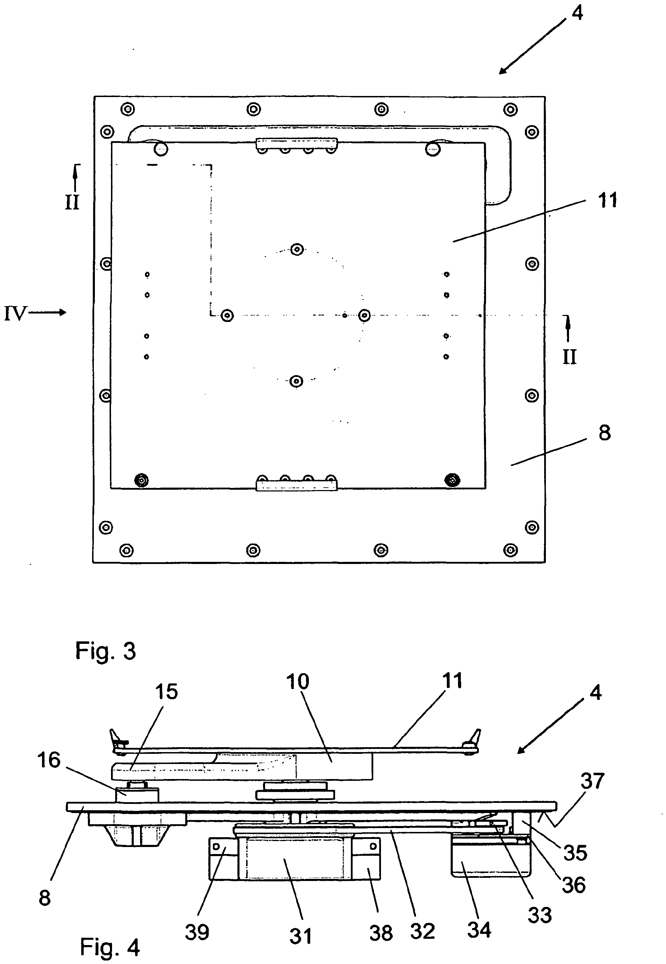

- Figur 2:: eine Seitenansicht einer Schüttelvorrichtung entlang der Linie II - II von Figur 3 geschnitten,
- Figur 3:: eine Draufsicht auf die Schüttelvorrichtung von Figur 2 und
- Figur 4:: eine Seitenansicht der Schüttelvorrichtung von Figur 3 aus Richtung IV,

Ein Inkubator 1 besteht im Wesentlichen aus einem Inkubationsraum 2, einem Vorrichtungsraum 3 und einer Schüttelvorrichtung 4.

In dem Inkubator 1 ist der Inkubationsraum 2, in dem in Gefäßen 5 befindliche Zellkulturen kultiviert werden, angeordnet. Zu dem Zweck der Kultivierung sind im Inkubationsraum 2 Temperatur und Feuchte einstellbar. Dem Inkubationsraum 2 ist ein Vorrichtungsraum 3 in vertikaler Richtung unten vorgelagert. Ein Boden 6 des Inkubationsraums 2 weist zum Vorrichtungsraum 3 hin eine Bodenöffnung 7 als Verbindung zum Vorrichtungsraum 3 auf.

Die Schüttelvorrichtung 4 ist als eine Einbauvorrichtung ausgebildet, die so in die Bodenöffnung 7 einsetzbar ist, dass ihre Grundplatte 8 den Inkubationsraum 2 gegenüber dem Vorrichtungsraum 3 abdichtet. Die Grundplatte 8 weist auf ihrer dem Inkubationsraum 2 zugewandten Innenseite 9 einen von einem Antriebsarm 10 in einer horizontalen Ebene beweglichen Schütteltisch 11 auf. Der Schütteltisch 11 ist über Schrauben 12 fest mit dem Antriebsarm 10 verbunden. Der Antriebsarm 10 ist einerseits über einen Achszapfen 20 mit einer Antriebsachse 14 und andererseits über zwei an radial abstehenden Enden 15 des Antriebsarms 10 angeordneten exzentrischen Drehgelenken 16 und deren Gelenkzapfen 25 und 29 mit der Grundplatte 8 verbunden.

Die Antriebsachse 14 ist über zwei hintereinander geschaltete Rillen- bzw. Kugellager 17 an der Grundplatte 8 gelagert. An ihrem in vertikaler Richtung oberen Ende weist die Antriebsachse 14 einen Aufnahmeansatz 18 für den Achszapfen 20 auf. Der Achszapfen 20 weist an seinem unteren Ende einen Flansch 19 auf, mit dem er mit dem Aufnahmeansatz 18 verschraubbar ist. In vertikaler Richtung oben ist der obere Achszapfen 20 über zwei Kugellager 21 im Antriebsarm 10 drehbar gelagert. Der obere Achszapfen 20 ist seitlich versetzt, parallel zur Längsachse 22 der Antriebsachse 14 angeordnet. Der Achszapfen 20 ist mit seinem Flansch 19 in mindestens zwei unterschiedlichen Abständen von der Längsachse 22 an dem Aufnahmeansatz 18 der Antriebsachse 14 befestigbar.
Die Antriebsachse 14 ist auf der Innenseite 9 gegenüber der Grundplatte 8 durch eine Dichtung 23 abgedichtet.

Die Drehgelenke 16 weisen ein unteres Gelenkteil 24 mit einem unteren Gelenkzapfen 25 auf, über den sie durch Kugellager 26 an der Grundplatte 8 drehbar gelagert sind. Über ein elastisches Dämpfungsglied 27 ist das untere Gelenkteil 24 mit einem oberen Gelenkteil 28 verbunden, das einen oberen Gelenkzapfen 29 aufweist, der seinerseits über ein Kugellager 26 in dem Antriebsarm 10 drehbar gelagert ist.

Der obere Gelenkzapfen 29 ist in mindestens zwei unterschiedlichen Abständen von der Längsachse 40 des unteren Gelenkzapfens 25 an dem oberen Gelenkteil 28 befestigbar. Dieser Abstand entspricht dem seitlichen Abstand des oberen Achszapfens 20 zur Längsachse 22 der Antriebsachse 14.

Die Antriebsachse 14 weist an ihrem dem Vorrichtungsraum 3 zugewandten Ende einen unteren Achszapfen 30 auf, an dem eine Schwungscheibe 31 angeordnet ist, die über einen Antriebsriemen 32 mit einem Antriebsrad 33 eines die Schüttelvorrichtung 4 antreibenden Motors 34 verbunden ist. Der Motor 34 ist über Trägersäulen 35 mit einer Befestigungsplatte 36 beabstandet zu der der Innenseite 9 abgewandten Außenseite 37 der Grundplatte 8 angeordnet.

Die Schwungscheibe 31 weist ein radial verschiebbares Kontergewicht 38 auf, das um ein Zusatzgewicht 39 ergänzbar ist.

## Patentansprüche

1. Inkubator (1) mit einem Inkubationsraum (2), einer von einem Antrieb antreibbaren Schüttelvorrichtung (4) zum Schütteln von in dem Inkubationsraum (2) anordenbaren Gefäßen (5) mit Zellkulturen, und einem dem Inkubationsraum (2) benachbarten Vorrichtungsraum (3) zur Aufnahme mindestens von Teilen der in den Inkubationsraum (2) hineinragenden Schüttelvorrichtung (4),
**dadurch gekennzeichnet,**
**dass** die Schüttelvorrichtung (4) eine Grundplatte (8) aufweist, die den Inkubationsraum (2) gegenüber dem Vorrichtungsraum (4) abdichtet und auf ihrer dem Inkubationsraum (2) zugewandten Innenseite (9) einen von einem Antriebsarm (10) in einer horizontalen Ebene beweglichen Schütteltisch (11) aufweist, und dass auf der der Innenseite (9) abgewandten Außenseite (37) der Grundplatte (8) ein Motor (34) angeordnet ist, über den eine in der Grundplatte (8) rotierend gelagerte Antriebsachse (14) antreibbar ist, die mit dem Antriebsarm (10) über zwei Achszapfen (20, 30) verbunden ist.

2. Inkubator nach Anspruch 1,
**dadurch gekennzeichnet,**
**dass** die Antriebsachse (14) an ihrem dem Vorrichtungsraum (4) zugewandten Ende eine Schwungscheibe (31) aufweist, die von dem Motor (34) antreibbar ist.

3. Inkubator nach Anspruch 2,
**dadurch gekennzeichnet,**
**dass** die Schwungscheibe (31) zentrisch an einem unteren Achszapfen (30) angeordnet und über einen Antriebsriemen (32) mit einem Antriebsrad (33) des Motors (34) verbunden ist.

4. Inkubator nach Anspruch 2 oder 3,
**dadurch gekennzeichnet,**
**dass** an der Schwungscheibe (31) ein radial verschiebbares Kontergewicht (38) angeordnet ist.

5. Inkubator nach Anspruch 3 oder 4,
**dadurch gekennzeichnet,**
**dass** die Antriebsachse (14) an ihrem dem Inkubationsraum (2) zugewandten Ende einen oberen Achszapfen (20) aufweist, der parallel versetzt zu dem unteren Achszapfen (30) angeordnet ist.

6. Inkubator nach einem der Ansprüche 1 bis 5,
**dadurch gekennzeichnet,**
**dass** der Antriebsarm (10) mit mindestens einem radial abstehenden Ende über ein exzentrisches Drehgelenk (16) an der Grundplatte (8) zusätzlich gelagert ist.

7. Inkubator nach Anspruch 6,
**dadurch gekennzeichnet,**
**dass** das exzentrische Drehgelenk (16) ein elastisches Dämpfungsglied (27) aufweist.

8. Inkubator nach Anspruch 7,
**dadurch gekennzeichnet,**
**dass** das untere Gelenkteil (24) über einen unteren Gelenkzapfen (25) an der Grundplatte (8) drehbar gelagert und über das elastische Dämpfungsglied (27) mit einem oberen Gelenkteil (28) verbunden ist, das einen oberen Gelenkzapfen (29) aufweist, der seinerseits in dem Antriebsarm 10 drehbar gelagert ist.

9. Inkubator nach Anspruch 8,
**dadurch gekennzeichnet,**
**dass** das der obere Gelenkzapfen (29) in mindestens zwei unterschiedlichen Abständen von der Längsachse (40) des unteren Gelenkzapfens (25) an dem oberen Gelenkteil (28) befestigbar ist.

10. Inkubator nach einem der Ansprüche 1 bis 9,
**dadurch gekennzeichnet,**
**dass** die Antriebsachse (14) über Lager (17) mit der Grundplatte (8) verbunden ist.

11. Inkubator nach einem der Ansprüche 1 bis 10,
**dadurch gekennzeichnet,**
**dass** zwischen der Antriebsachse (14) und der Grundplatte (8) eine Dichtung (23) angeordnet ist.

12. Inkubator nach einem der Ansprüche 1 bis 11,
**dadurch gekennzeichnet,**
**dass** die Dichtung (23) als eine reibungsarme Lippen- oder Labyrinthdichtung ausgebildet ist.

## Claims

1. Incubator (1) with an incubation chamber (2), a shaker device (4), which is drivable by a drive, for shaking flasks (5), which can be arranged in the incubation chamber (2), with cell cultures, and a device chamber (3), which is adjacent to the incubation chamber (2), for receiving at least parts of the shaker device (4) protruding into the incubation chamber (2), **characterised in that** the shaker device (4) comprises a base plate (8) which seals off the incubation chamber (2) relative to the device chamber (4) and has on its inner side (9) facing the incubation chamber (2) a shaking table (11) movable by a drive arm (10) in a horizontal plane, and that arranged on the outer side (37) of the base plate (8) remote from the inner side (9) is a motor (34) by way of which a drive axle (14) rotatably mounted in the base plate (8) and connected with the drive arm (10) by way of two axle pins (20, 30) is drivable.

2. Incubator according to claim 1, **characterised in that** the drive axle (14) has at its end facing the device chamber (4) a flywheel (31) drivable by the motor (34).

3. Incubator according to claim 2, **characterised in that** the flywheel (31) is arranged centrally at a lower axle pin (30) and is connected with a drive wheel (33) of the motor (34) by way of a drive belt (32).

4. Incubator according to claim 2 or 3, **characterised in that** a radially displaceable counterweight (38) is arranged at the flywheel (31).

5. Incubator according to claim 3 or 4, **characterised in that** the drive axle (14) has at its end facing the incubation chamber (2) an upper axle pin which is arranged to be parallel to and offset from the lower axle pin (30).

6. Incubator according to any one of claims 1 to 5, **characterised in that** the drive arm (10) is additionally mounted on the base plate (8) by at least one radially projecting end by way of an eccentric swivel joint (16).

7. Incubator according to claim 6, **characterised in that** the eccentric swivel joint (16) comprises a resilient damping element (27).

8. Incubator according to claim 7, **characterised in that** the lower swivel joint (24) is rotatably mounted on the base plate (8) by way of a lower swivel pin (25) and is connected by way of the resilient damping element (27) with an upper swivel part (28), which has an upper swivel pin (29) which in turn is rotatably mounted in the drive arm (10).

9. Incubator according to claim 8, **characterised in that** the upper pivot pin (29) is fastenable to the upper swivel part (28) at at least two different spacings from the longitudinal axis (40) of the lower swivel pin (25).

10. Incubator according to any one of claims 1 to 9, **characterised in** the drive axle (14) is connected with the base plate (8) by way of bearings (17).

11. Incubator according to any one of claims 1 to 10, **characterised in that** a seal (23) is arranged between the drive axle (14) and the base plate (8).

12. Incubator according to any one of claims 1 to 11, **characterised in that** the seal (23) is constructed as a low-friction lip seal or labyrinth seal.

## Revendications

1. Incubateur (1) comprenant une chambre d'incubation (2), un dispositif agitateur (4), pouvant être entraîné par un entraînement, pour l'agitation de récipients (5) comportant des cultures cellulaires disposés dans la chambre d'incubation (2), et une chambre pour le dispositif (3), attenante à la chambre d'incubation (2), pour au moins l'installation de pièces composant le dispositif d'agitation (4) pénétrant à l'intérieur de la chambre d'incubation (2)
**caractérisé en ce**
**que** le dispositif d'agitation (4) présente une plaque de base (8), qui rend la chambre d'incubation (2) étanche vis-à-vis de la chambre pour le dispositif (4), et qui présente une table d'agitation (11) mobile dans un plan horizontal par l'intermédiaire d'un bras d'entraînement (10) situé sur la face interne (9) de la chambre d'incubation (2), et qu'un moteur (34) est disposé sur la face externe (37) de la plaque de base (8) correspondant à la face interne (9), moteur par lequel un axe d'entraînement (14) peut être entraîné, logé, pouvant tourner dans la plaque de base (8), qui est relié avec le bras d'entraînement (10) par l'intermédiaire de deux tenons axiaux (20, 30).

2. Incubateur selon la revendication 1,
**caractérisé en ce**
**que** l'axe d'entraînement (14) présente un disque d'inertie (31) à l'extrémité orientée vers la chambre pour le dispositif (4), disque qui peut être entraîné par le moteur (34).

3. Incubateur selon la revendication 2
**caractérisé en ce**
**que** le disque d'inertie (31) est disposé de manière centrale sur l'un des tenons axiaux (30) et est relié avec une roue d'entraînement (33) du moteur (34) par l'intermédiaire d'une courroie d'entraînement (32).

4. Incubateur selon les revendications 2 ou 3
**caractérisé en ce**
**qu'**un contrepoids (38) pouvant être déplacé radialement est disposé sur le disque d'inertie (31).

5. Incubateur selon les revendications 3 ou 4
**caractérisé en ce**
**que** l'axe d'entraînement (14) présente un tenon axial supérieur (20) à son extrémité orientée vers la chambre d'incubation (2) qui est disposé de manière décalée parallèlement au tenon axial inférieur (30).

6. Incubateur selon l'une des revendications de 1 à 5
**caractérisé en ce**
**que** le bras d'entraînement (10) est logé complémentairement avec au moins une extrémité radiale écartée sur une articulation de rotation (16) excentrique sur la plaque de base (8).

7. Incubateur selon la revendication 6
**caractérisé en ce**
**que** l'articulation excentrique (16) présente un organe d'amortissement (27) élastique.

8. Incubateur selon la revendication 7
**caractérisé en ce**
**que** la partie d'articulation inférieure (24) est logée, pouvant tourner, sur un tenon d'articulation (25) inférieur sur la plaque de base (8) et est reliée avec une partie d'articulation supérieure (28) par l'intermédiaire de l'organe d'amortissement (27) élastique, la partie supérieure qui présente un tenon d'articulation supérieur (29), qui, pour sa part, est logé, pouvant être mis en rotation, dans le bras d'articulation 10.

9. Incubateur selon la revendication 8
**caractérisé en ce**
**que** le tenon d'articulation supérieur (29) peut être fixé sur la partie d'articulation supérieure (28) sur au moins deux espaces différents de l'axe longitudinal (40) du tenon d'articulation (25) inférieur.

10. Incubateur selon l'une des revendications de 1 à 9
**caractérisé en ce**
**que** l'axe d'entraînement (14) est relié avec la plaque de base (8) par l'intermédiaire de paliers (17).

11. Incubateur selon l'une des revendications de 1 à 10
**caractérisé en ce**
**qu'**un joint (23) est disposé entre l'axe d'entraînement (14) et la plaque de base (8).

12. Incubateur selon l'une des revendications de 1 à 11
**caractérisé en ce**
**que** le joint (23) est conçu comme un joint à lèvres ou un joint labyrinthe présentant peu de friction.
